# FASCICULE DE BREVET EUROPEEN

(11) **EP 2 697 383 B1**
(45) Date de publication et mention de la délivrance du brevet: **17.10.2018**
(21) Numéro de dépôt: 12714746.0
(22) Date de dépôt: 27.03.2012
(51) Int. Cl.: C12P 7/10, C12P 7/14

(54) **Procédé de production d'éthanol à partir de biomasse cellulosique ou lignocellulosique avec recyclage d'un vin éthylique issu de la fermentation des pentoses**
Verfahren zur Herstellung von Ethanol aus cellulose- oder lignocellulosehaltiger Biomasse mit Rückführung einer ethanolhaltigen Fermentationsbrühe aus der Fermentation der Pentosefraktion
Method for producing ethanol from cellulosic or lignocellulosic biomass including the recirculation of an ethylic wine obtained by fermentation of pentoses

(30) Priorité: 14.04.2011 FR 1101147
(43) Date de publication de la demande: 19.02.2014
(73) Titulaire: IFP Energies nouvelles, 92500 Rueil-Malmaison (FR)
(72) Inventeur: ROPARS, Marcel, F-91120 Palaiseau (FR); AYMARD, Caroline, F-69002 Lyon (FR); DASTILLUNG, Réjane, 69005 Lyon (FR); MENIR, Sandra, F-95500 Gonesse (FR)
(74) Mandataire: IFP Energies nouvelles
(86) Numéro de dépôt international: PCT/FR2012/000108
(87) Numéro de publication internationale: WO 2012/140332

(56) Documents cités:
- WO-A2-2007/009463
- WO-A2-2010/130888
- US-A1- 2009 093 027
- US-A1- 2010 159 552
- ABHIJIT DUTTA ET AL: "An economic comparison of different fermentation configurations to convert corn stover to ethanol using Z. mobilis and Saccharomyces", BIOTECHNOLOGY PROGRESS, vol. 26, no. 1, 1 janvier 2009 (2009-01-01), pages 64-72, XP055014122, ISSN: 8756-7938, DOI: 10.1002/btpr.311
- HAMELINCK C N ET AL: "Ethanol from lignocellulosic biomass: techno-economic performance in short-, middle- and long-term", BIOMASS AND BIOENERGY, PERGAMON, vol. 28, no. 4, 1 avril 2005 (2005-04-01), pages 384-410, XP027829269, ISSN: 0961-9534 [extrait le 2005-04-01]

## Description

### DOMAINE DE L'INVENTION

La présente invention s'inscrit dans le cadre d'un procédé de production d'alcools et/ou de solvants dit de "seconde génération" à partir de biomasse lignocellulosique. Elle concerne plus particulièrement un procédé de production d'éthanol à partir de biomasse cellulosique ou lignocellulosique.

### ART ANTÉRIEUR

La biomasse lignocellulosique représente une des ressources renouvelables les plus abondantes sur terre. Les substrats considérés sont très variés, puisqu'ils concernent à la fois les substrats ligneux (feuillus et résineux), les sous-produits de l'agriculture (paille) ou ceux des industries génératrices de déchets lignocellulosiques (industries agroalimentaires, papeteries).

La biomasse lignocellulosique est composée de trois principaux polymères : la cellulose (35 à 50%), l'hémicellulose (20 à 30%) qui est un polysaccharide essentiellement constitué de pentoses et d'hexoses et la lignine (15 à 25%) qui est un polymère de structure complexe et de haut poids moléculaire, composé d'alcools aromatiques reliés par des liaisons éther.

Ces différentes molécules sont responsables des propriétés intrinsèques de la paroi végétale et s'organisent en un enchevêtrement complexe.

La cellulose et éventuellement les hémicelluloses sont les cibles de l'hydrolyse enzymatique mais ne sont pas directement accessibles aux enzymes. C'est la raison pour laquelle ces substrats doivent subir un prétraitement précédant l'étape d'hydrolyse enzymatique. Le prétraitement vise à modifier les propriétés physiques et physico-chimiques du matériau lignocellulosique, en vue d'améliorer l'accessibilité de la cellulose emprisonnée au sein de la matrice de lignine et d'hémicellulose.

De nombreuses technologies pour réaliser ce prétraitement existent : cuissons acides, cuissons alcalines, explosion à la vapeur, procédés organosolv, etc. L'efficacité du prétraitement se mesure à la fois par le bilan matière à l'issue du prétraitement (taux de récupération des sucres sous forme de monomères ou oligomères solubles ou polymères insolubles) et également par la susceptibilité à l'hydrolyse enzymatique des résidus cellulosiques et hémicellulosiques.

Les procédés de production d'alcools et/ou de solvants à partir de biomasse lignocellulosique, dit "procédés de seconde génération" comprennent au moins les étapes suivantes :
- prétraitement du substrat,
- hydrolyse enzymatique du substrat prétraité,
- fermentation de l'hydrolysat obtenu , et
- séparation /purification de l'alcool et/ou solvant obtenu après fermentation.

La validité économique de ce type de procédé de production d'alcool et/ou de solvant est difficile à obtenir même pour les opérateurs disposant d'une large ressource mobilisable. Plusieurs postes ont un fort impact sur le coût global dont la ressource végétale et l'énergie pour l'extraction le plus souvent réalisée par distillation. L'optimisation de ce type de procédé passe obligatoirement par une valorisation optimale de l'ensemble des sucres, et notamment les pentoses issus de l'hydrolyse par les microorganismes les mieux adaptés

Les levures alcooligènes sauvages telles que *Saccharomyces cerevisiae* sont connues pour être les microorganismes les plus efficaces pour la conversion des hexoses en éthanol. Les rendements massiques de conversion des hexoses en éthanol sont en général compris entre 0,46 et 0,48, mais ne sont que de 0,35 à 0,40 pour la conversion des pentoses en éthanol. Ces levures sauvages ne sont pas capables de convertir les pentoses sans une modification génétique. L'usage de microorganismes génétiquement modifiés complique la gestion des installations et celle du procédé. Ainsi, les levures modifiées vont toujours utiliser prioritairement les hexoses et elles ne peuvent utiliser ensuite ces pentoses qu'en présence d'une quantité limitée de glucose qu'il convient de fournir par une alimentation continue au microorganisme. Il est connu que la vitesse de consommation des pentoses est nettement inférieure à celle des hexoses (Olsson et Hahn-Hagerdahl, 1996; Hahn-Hägerdal et al., 2007).

La valorisation des pentoses en éthanol a toujours constitué un problème majeur pour le procédé dans son ensemble. Si certaines levures sauvages sont capables de convertir ces pentoses en éthanol, elles doivent obligatoirement être cultivées en micro aérobiose pour avoir des performances satisfaisantes. Par ailleurs, il est préférable que le milieu soit essentiellement constitué de pentoses, car ces levures présentent des performances nettement inférieures à celles de *Saccharomyces cerevisiae* pour la valorisation des hexoses (Olsson et Hahn-Hagerdahl, Enzymes Microb. Techno vol 18, 1996; Hahn-Hägerdal et al., Appl. Microbiol. Biotech 74, 937-953, 2007)

Le document de ABHIJIT DUTTA ET AL, "An economic comparison of différent fermentation configurations to convert corn stover to ethanol using Z. mobilis and Saccharomyces", BIOTECHNOLOGY PROGRESS, (20090101), vol. 26, no. 1, pages 64 - 72 décrit et compare différentes confifurations de procédé de production d'éthanol à partir de biomasse lignocellulosique.

D'autres solutions sont envisageables avec la mise en oeuvre de levures génétiquement modifiées qui sont capables d'utiliser correctement le xylose, après avoir consommé le glucose ou en le consommant de façon concomitante. Toutefois, ces types de levures ont généralement besoin d'une injection limitée et contrôlée du glucose pour assurer la consommation du xylose, ce qui complique très nettement le contrôle du procédé. Par ailleurs, ces organismes génétiquement modifiés doivent être cultivés dans des conditions strictes de confinement, qui sont peu compatibles avec une production de masse dans les installations de très grandes tailles et rustiques.

D'autre part, l'extraction des alcools réalisée par distillation est un poste particulièrement énergivore. Pour l'amélioration du bilan économique de la production d'éthanol, il convient d'envisager de réduire les volumes d'eau qui doivent être chauffés notamment au moyen de recyclages ou regroupements de différents flux qui concentrent les solvants.

La présente invention décrit un procédé de production d'éthanol dans lequel une partie du vin éthylique produit principalement à partir des seuls pentoses, est recyclée au sein de la ligne de conversion des hexoses en éthanol pour réduire le coût global de l'extraction.

### RÉSUMÉ DE L'INVENTION

La présente invention porte sur un procédé de production d'éthanol dit de seconde génération, dans lequel la biomasse lignocellulosique ou cellulosique subit un prétraitement, avant d'être convertie en éthanol après une hydrolyse enzymatique et une fermentation éthylique. Les polymères glucidiques du végétal prétraité sont hydrolysés par des cellulases. Les microorganismes alcooligènes utilisées pour la fermentation éthylique utilisent principalement les hexoses et de préférence les glucose et mannose. L'éthanol du vin, avec ou sans séparation des matières en suspension (MES), sont extraits par distillation. Les vinasses contiennent principalement les pentoses qui n'ont pas été utilisés. Ces pentoses sont fermentés en un vin éthylique ex-pentoses et au moins une partie de ce vin ainsi obtenu est recyclée en amont de l'hydrolyse enzymatique. L'hydrolyse enzymatique et la fermentation éthylique sont donc réalisées, séparément ou simultanément, en présence d'une partie du vin éthylique, mélange qui ne gêne pas les performances des enzymes et des microorganismes dans les conditions opérées.

### DESCRIPTION DES DESSINS

La Figure 1 est une représentation schématique d'un procédé de production d'éthanol à partir de substrats lignocellulosiques, comprenant une étape de recyclage du vin éthylique ex-pentoses, selon un premier mode de réalisation.
La Figure 2 est une représentation schématique d'un procédé de production d'éthanol à partir de substrats lignocellulosiques, comprenant une étape de recyclage du vin éthylique ex-pentoses, selon un second mode de réalisation.

### DESCRIPTION DÉTAILLÉE DE L'INVENTION

La présente invention décrit un procédé de production d'éthanol à partir de biomasse cellulosique ou lignocellulosique selon la revendication 1.

Au sens de la présente invention, on désigne sous le terme de "pentoses" les monomères et oligomères solubles des sucres comprenant 5 atomes de carbone et sous le terme de "hexoses" les monomères et oligomères solubles des sucres comprenant 6 atomes de carbone.

On désigne sous l'abréviation MS les matières sèches (solides et solubles) présentes dans un milieu et sous l'abréviation MES les matières en suspension (solides) présentes dans un milieu.

On désigne sous le terme vin éthylique ex-hexoses le vin issu de la fermentation par des microorganismes fermentant les hexoses.

On désigne sous le terme vin éthylique ex-pentoses le vin issu de la fermentation par des microorganismes fermentant les pentoses .

Grâce au procédé selon la présente invention, il est possible d'améliorer nettement le bilan énergétique de l'étape d'extraction. En effet, les vins éthyliques issus respectivement de la fermentation des hexoses et des pentoses étant regroupés, une seule étape d'extraction est nécessaire. L'économie est environ de 5% à 50%.

Avantageusement, cette étape d'extraction est réalisée sur un vin de titre alcoolique plus élevé, la conversion des hexoses étant opérée dans le milieu contenant l'éthanol issu de la fermentation des pentoses.

Le procédé selon l'invention utilise de façon préférée, des microorganismes différents dans les étapes d) et f), les uns étant adaptés à la fermentation des hexoses, les autres étant capables de fermenter les seuls pentoses, de préférence sans un apport continu et limitant de glucose.

Le substrat cellulosique ou lignocellulosique utilisé dans le procédé selon la présente invention est choisi parmi les biomasses les plus variées, mais plus particulièrement depuis les espèces arborescentes résineuses (softwood tels que les épicéas ou les pins) ou feuillues (hardwood tels que les eucalyptus) ou les déchets lignocellulosiques agricoles (paille de blé, riz, etc.) ou encore les cultures dédiées (miscanthus, switchgrass).

Préalablement au prétraitement thermo-chimique, la biomasse peut subir un traitement mécanique, par exemple de type broyage.

Sous le terme de prétraitement thermo-chimique, on comprendra tout prétraitement connu de l'homme du métier mettant en oeuvre des agents chimiques, alcalins ou acides, et/ou un chauffage de la biomasse.

Le prétraitement réalisé à l'étape a) peut être réalisé selon de nombreuses configurations connues de l'homme du métier (Hendriks et Zeeman, Bioressource Technology, 100(2009) 10-18 ; Ogier et al, Oil & Gas Science and Technology, vol 54 (1999) p 67-94). On peut citer le prétraitement alcalin par cuisson en présence de soude, un prétraitement par explosion des fibres à l'ammoniac, encore appelé prétraitement AFEX (Ammonia Fiber Explosion) ou un prétraitement par percolation utilisant de l'ammoniaque avec recycle, encore appelé prétraitement ARP (Ammonia Recycle Percolation). On peut également citer les cuissons acides ou l'explosion à la vapeur en conditions acides.

Préférentiellement, le prétraitement de l'étape a) est une cuisson acide ou une explosion à la vapeur en conditions acides.

Le rôle du prétraitement est de rendre la cellulose accessible aux enzymes, en déstructurant la matrice lignocellulosique. En fonction du prétraitement réalisé, on attaque préférentiellement la lignine, les hémicelluloses ou les deux à la fois.

Des étapes additionnelles de mise au pH ou de liquéfaction peuvent être réalisées afin de faciliter la mise en oeuvre et l'efficacité du procédé et notamment le déroulement des étapes de l'hydrolyse enzymatique et de la fermentation éthylique

La conversion de la cellulose en éthanol comprend au moins une étape d'hydrolyse enzymatique de la cellulose en glucose et une étape de fermentation du glucose en éthanol, ces deux étapes pouvant être réalisées séparément ou simultanément. Lorsque les deux étapes sont opérées simultanément, le procédé est appelé "procédé SSF".

Les enzymes cellulolytiques et/ou hemicellulolytiques utilisées pendant l'étape d'hydrolyse sont produites par un microorganisme appartenant aux genres *Trichoderma, Aspergillus, Penicillium* ou *Schizophyllum,* ou une bactérie anaérobie appartenant au genre *Clostridium.*

L'hydrolyse est préférentiellement réalisée à pH compris entre 4 et 5,5 et à une température entre 40°C et 60°C.

La fermentation éthylique réalisée à l'étape d)_est assurée par des levures ou autres microorganismes alcooligènes.

Les microorganismes alcooligènes utilisés pendant l'étape de fermentation éthylique des hexoses sont de préférence choisis parmi les levures et les bactéries, éventuellement génétiquement modifiées.

Lorsque le microorganisme alcooligène est une levure, *Saccharomyces cerevisiae* est celle qui est la plus performante. Il est également possible de choisir des levures telles que *Schizosaccharomyces pombe ou Saccharomyces uvarum ou diastaticus.* Des levures plus thermophiles, telles que les *Kluyveromyces fragilis* (maintenant souvent désignée par K. *marxianus*) présentent également un intérêt, notamment lorsque l'hydrolyse enzymatique et la fermentation éthylique sont réalisées simultanément (procédé SSF).

Un organisme génétiquement modifié, comme par exemple une levure de type *Saccharomyces cerevisiae* telle que la TMB 3400 (Ohgren et al, J. of Biotech 126, 488-498, 2006) peut également être utilisé. Cette levure permet de fermenter en éthanol une partie des pentoses lors de l'étape de fermentation éthylique des hexoses, lorsque le glucose est en concentration limitante.

Lorsque le microorganisme alcooligène est une bactérie, on préférera *Zymomonas mobilis* qui présente une voie d'assimilation efficace.

La fermentation éthylique des hexoses est réalisée préférentiellement à une température comprise entre 30°C et 40°C, et un pH entre 3 et ,6,5.

Les levures, et de préférence *Saccharomyces cerevisiae* sont les microorganismes utilisés de façon très préférée. Ils présentent une meilleure robustesse, sécurité, et ne nécessitent pas de stérilité pour la conduite du procédé et des installations.

Les levures du genre *Saccharomyces* sont capables de fermenter les seuls et uniques hexoses (glucose et mannose essentiellement). Ces levures valorisent de façon optimale les hexoses en éthanol et permettent d'atteindre des rendements de conversion de l'ordre de 0,46 (p/p) à 0,48 (p/p), ce qui est proche du rendement théorique maximal qui vaut 0,51 (p/p). Seuls les pentoses et quelques sources carbonées marginales ne sont pas utilisés par ces levures.

Lorsque l'hydrolyse enzymatique et la fermentation éthylique des hexoses sont réalisées dans une même et seule opération (SSF), cette opération est préférentiellement réalisée à une température comprise entre 30 et 45°C, et à pH compris entre 4 et 6.

Lors de l'étape e1), l'éthanol produits à l'étape d) ou présents à l'étape d) suite à un recyclage du vin éthylique ex-pentoses sont purifiés et séparés. Ils sont ensuite séparés par toute méthode connue de l'homme de l'art, et en particulier par distillation.

Lors de l'étape e2) un gâteau solide contenant le résidu insoluble est séparé des vinasses contenant les sucres non fermentés par le microorganisme alcooligène. Les vinasses contiennent donc les pentoses non fermentés.

L'étape e2) peut être réalisée en aval des étapes c) et/ou d) et peut éventuellement être couplée à un lavage du gâteau. Le lavage permet d'améliorer la récupération des sucres issus de l'hydrolyse (étape c), de l'éthanol produits lors de l'étape d) ou présents du fait du recyclage et/ou également des sucres non fermentés par le microorganisme lors de l'étape d).

Lors de l'étape f) de fermentation éthylique des pentoses f) par un microorganisme fermentant plus spécifiquement les pentoses, les sucres contenus dans les vinasses et notamment les pentoses non fermentés, sont également convertis en un vin éthylique ex-pentoses.

Les microorganismes utilisés pour la fermentation des pentoses peuvent être des bactéries, des levures ou des champignons.

Parmi les bactéries, on peut citer les bactéries "sauvages" appartenant aux genres *Bacillus, Bacteroides, Thermoanaerobacter ou Clostridium,* des bactéries recombinantes dont les plus intéressantes sont *Escherichia coli, Klebsiella oxytoca et Zymomonas mobilis.*

Des levures sauvages telles que *Pichia stipitis, Candida shehatae* et *Pachysolen tannophilus,* identifiées comme étant les plus efficaces, peuvent être utilisées. Parmi les autres levures sauvages mentionnées dans la littérature peuvent être citées *Candida guilliermondii ou Candida tropicalis.*

Il est possible de choisir des levures recombinantes, notamment les *Saccharomyces cerevisiae,* telles que décrites par Olsson et Hahn-Hagerdahl, 1996 ou Hahn-Hagerdahl et al. 2007 .

Un organisme génétiquement modifié, comme par exemple une levure de type *Saccharomyces cerevisiae* telle que la TMB 3400 (Ohgren et al, 2006) peut également être utilisé pour la conversion des pentoses en éthanol, à condition de leur fournir du glucose en conditions limitantes pour permettre l'assimilation des pentoses.

Des champignons sont également capables de produire de l'éthanol dont de nombreux *Fusarium,* mais tous avec de plus faibles performances.

De façon préférée, on utilise des levures choisies parmi les genres *Pichia ou Candida.* Il s'agit de microorganismes plus difficiles à cultiver que la levure *Saccharomyces cerevisiae* car elles ont besoin d'oxygène pour avoir des performances acceptables (conditions de "micro-aérobies", Fromanger et al., J. Ind. Microbiol. Biotechnol, 37, 437-445, 2010) Hahn-Hägerdal et al., 2007). Ces levures fermentant les pentoses sont également connues pour être sensibles aux composés inhibiteurs qui altèrent les performances (Girio et al., Biores Technol, 101, 4775-4800, 2010).

Dans le procédé selon la présente invention, ces levures fermentant les pentoses sont avantageusement utilisées sur un milieu qui a partiellement été détoxifié par la levure fermentant les hexoses, par exemple par *Saccharomyces cerevisiae,* connue pour réduire notamment les principaux aldéhydes (Ohgren et al., Applied Biochemistry and Biotechnology vol 121-124, 1055-1067 (2005) ; Klinke et al., Appl Microbiol Biotechnol 66: 10-26, 2004) tels que les furfural et 5-HMF

Ceci est particulièrement avantageux dans le cas des hydrolysats lignocellulosiques issus de prétraitements réalisés en conditions acide comme l'explosion à la vapeur (en présence d'acide sulfurique ou SO2) ou la cuisson avec de l'acide dilué (acide sulfurique). Ces prétraitements aboutissent en général à la production de composés furaniques, phénoliques et des acides organiques qui sont connus pour être toxiques pour les microorganismes.

De façon préférée, la souche utilisée dans l'étape de fermentation f) est une souche de type *Pichia stipitis ou Candida shehatae.*

De façon préférée, les vinasses, qui sont envoyées vers l'étape f) de fermentation éthylique des pentoses ont une concentration en sucres comprise entre 30 et 90 g/L de sucres ou produits fermentescibles.

Si la concentration en sucres ou produits fermentescibles n'est pas suffisante, un mode de réalisation particulier permet de dévier ce flux en aval de l'étape de prétraitement avant de l'envoyer vers l'étape de fermentation f). Ceci permet une mise en suspension des matières insolubles et un enrichissement en pentoses et sucres non utilisés par la fermentation éthylique.

Selon un autre mode, les pentoses fermentés à l'étape f) proviennent à la fois des vinasses et d'un flux directement extrait après l'étape de prétraitement lorsque celui ci est acide.

Selon cette variante du procédé, un flux contenant majoritairement des pentoses est extrait directement lors du prétraitement et est envoyé vers l'étape de fermentation éthylique des pentoses. Cette variante est applicable lorsque le prétraitement est acide et comporte une hydrolyse chimique des hémicelluloses. C'est notamment le cas lorsque le prétraitement est une explosion à la vapeur, réalisée en conditions acides. Il est alors possible d'obtenir en sortie de l'étape de prétraitement un jus de pentoses convertible en solvants, par exemple en ABE ou utilisable pour une autre application, s'il n'est pas envoyé en totalité vers l'étape de fermentation f). Cette possibilité permet notamment d'augmenter le taux de matière sèche du végétal prétraité soumis à l'hydrolyse enzymatique et à la fermentation éthylique des hexoses, voire de détoxifier partiellement ce flux en retirant au moins une partie des inhibiteurs avec le jus de pentoses.

De préférence, la concentration en éthanol à l'issue de l'étape de fermentation éthylique ex-pentoses f) est comprise entre 5 et 40g/L, et de façon préférée entre 5 et 30 g /L.

Selon l'invention, au moins une partie du vin éthylique ex-pentoses est recyclée en aval de l'étape a) de prétraitement.

L'inactivation du microorganisme ayant fermenté les pentoses est effectuée soit par une augmentation de la température soit par une modification du pH, ou de toute autre technique connue de l'homme de l'art. La modification du pH est préférée dans la mesure où il peut être nécessaire de corriger le pH pour l'étape d'hydrolyse enzymatique (étape b), après le prétraitement (étape a).

Selon un mode de réalisation, le flux contenant le vin éthylique ex-pentoses sortant de l'étape de fermentation éthylique des pentoses est divisé en deux flux, l'un étant recyclé en aval de l'étape de prétraitement.

Les vins éthyliques ex-hexoses et ex-pentoses soumis à l'extraction (étape e) peuvent contenir entre 20 et 150 g/L d'éthanol. La concentration en éthanol dépend d'une part de la teneur en matière sèche, des étapes d'hydrolyse enzymatique et de fermentation et d'autre part des éventuels compléments en sucre qu'il est possible d'apporter. On peut envisager de complémenter le milieu par du sucre de canne à sucre ou de betteraves sucrières ou de plantes amylacées.

L'invention va être décrite de façon détaillée en se référant aux figures.

Le substrat est introduit par la conduite 1 dans le réacteur de prétraitement 2. Les réactifs et utilités telles que la vapeur nécessaires à la conduite du prétraitement sont introduits par la canalisation 3 et les résidus (condensats, liqueur noire, eaux de lavage, ...) extraits par la conduite 4. Le recyclage, la réutilisation ou le traitement de ce flux est inhérent à chaque type de prétraitement et n'est pas détaillé ici.

Le substrat prétraité est extrait par la conduite 6. Il contient préférentiellement entre 5%(p/v) et 60%(p/v) de MS, plus préférentiellement entre 15%(p/v) et 60%(p/v) de MS et encore plus préférentiellement entre 30%(p/v) et 60%(p/v) de MS.

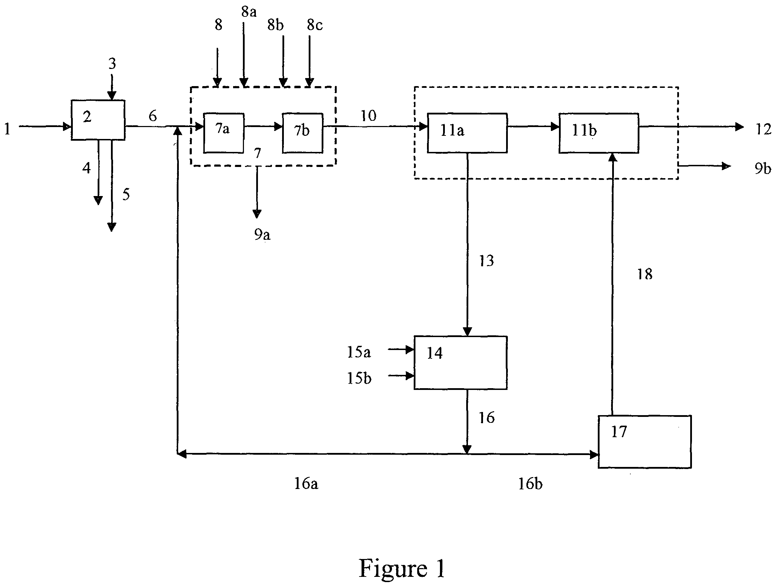

Ainsi, selon le mode de réalisation représenté sur la Fig.1, le substrat prétraité extrait par la conduite 6 contient la majorité des pentoses, sous forme solide (pentosanes) ou soluble.

Selon une variante du procédé dans laquelle le prétraitement est de type acide, un flux 5 contenant majoritairement des pentoses est extrait directement lors du prétraitement et est envoyé vers le réacteur 14 dans lequel a lieu la fermentation éthylique des pentoses.

Selon une autre variante non représentée, les pentoses peuvent être extraits en partie dans le flux 5 et en partie dans le substrat prétraité circulant dans la conduite 6, et ces deux flux mélangés tout ou en partie avant la fermentation éthylique des pentoses .

Le réacteur 7 est le réacteur dans lequel on réalise la conversion de la cellulose en éthanol.

Les conditions de l'hydrolyse enzymatique, principalement le taux de matière sèche du mélange à hydrolyser et la quantité d'enzymes utilisée, sont choisies de telle façon que l'étape c) soit réalisée pour pouvoir obtenir une solubilisation de la cellulose comprise entre 20% et 99% au sein du réacteur 7, et plus particulièrement entre 30% et 95%. L'eau nécessaire à l'obtention du taux de MS visé est ajoutée par la conduite 8. Le taux de MS souhaité est compris entre 5%(p/v) et 45%(p/v) et préférentiellement entre 8%(p/v) et 35%poids.

Les enzymes cellulolytiques et/ou hémicellulolytiques sont ajoutées par la canalisation 8a.

Les microorganismes utilisés pour la fermentation éthylique des hexoses sont introduits par la canalisation 8b.

Les additifs nécessaires pour une mise au pH ou une liquéfaction sont introduits par la canalisation 8c.

L'extraction de l'éthanol produits lors de l'étape de fermentation éthylique est réalisée dans le réacteur 11. L'alcool est extraits préférentiellement par distillation, par la conduite 12.

Le gâteau contenant le résidu insoluble est extrait par les canalisations (9a) et/ou (9b).

Dans le réacteur 11a, on opère à la séparation de l'éthanol et les vinasses sont extraites par la canalisation 13.

Dans le réacteur 11b, on réalise plus spécifiquement la séparation entre l'éthanol et l'eau pour extraire un flux de produits par la canalisation 12.

Ainsi, en sortie des étapes c) à e) réalisées dans les réacteurs 7 et 11, on obtient un flux de produits 12 (éthanol) extrait par tout moyen connu de l'homme de l'art, un résidu liquide 13 (appelé vinasses) contenant des sucres non fermentés avec en particulier les pentoses (xylose, arabinose), voire des traces d'hexoses (galactose, par exemple, l'hexose le plus difficile à métaboliser par les levures conventionnelles) ainsi que des oligomères et un gâteau solide contenant de la matière solide issue du substrat initial (résidu solide) et une fraction liquide, du fait des limitations des équipements de séparation solide/liquide. Le résidu solide est en partie composé de cellulose et hémicellulose qui n'ont pas été hydrolysées et de lignine.

Le microorganisme utilisé lors de l'étape de fermentation éthylique des pentoses, est introduit dans le réacteur 14 par la conduite 15a pour être mélangé avec la vinasse, fraction 13. Les utilités et additifs nécessaires à une bonne conduite de la fermentation sont introduits par la conduite 15b. Le réacteur 14 peut être un réacteur stérilisable. Les gaz de fermentation sont évacués. Le pH dans ce réacteur peut être contrôlé et régulé si besoin.

Le flux sortant du réacteur 14 par la canalisation 16 correspond au vin éthylique ex-pentoses. Il n'est pas utile de séparer les microorganismes.

Le flux aqueux entrant dans le réacteur d'hydrolyse et/ou de fermentation des hexoses contient entre 5 et 40g/L d'éthanol.

Selon le mode de réalisation représenté sur la Figure 1, le flux 16 sortant de l'étape de fermentation éthylique des pentoses est divisé en deux flux. Le flux 16a est recyclé en aval de l'étape de prétraitement.

Le flux 16b peut être envoyé vers une étape de séparation eau/solvants dans un réacteur 17, avant d'être également recyclé et renvoyé vers le réacteur de séparation 11b via une conduite 18 .

Selon un autre mode de réalisation, le flux 16b est utilisé pour d'autres applications sans recyclage.

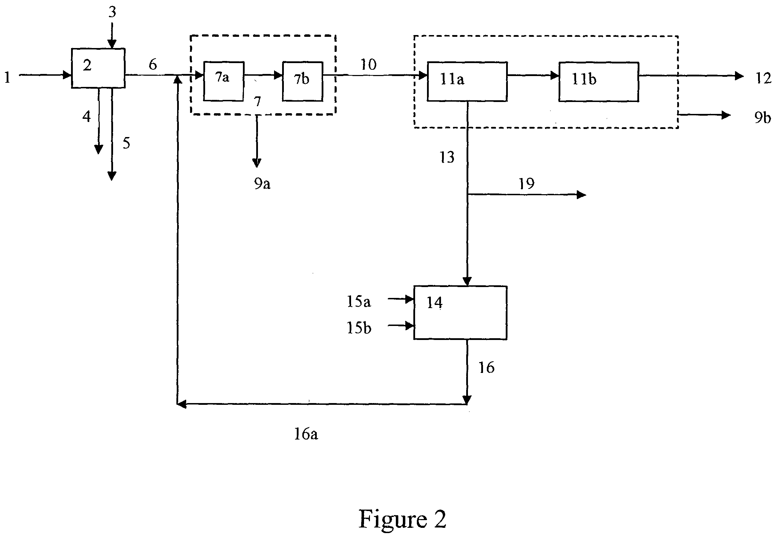

Selon le mode de réalisation représenté sur la Figure 2, la totalité du flux 16 sortant du réacteur de fermentation éthylique des pentoses est recyclé en aval de l'étape de prétraitement. La partie des vinasses en excès qui n'est pas envoyée vers l'étape f) de fermentation est extraite par la canalisation 19. Les sucres contenus dans ces vinasses peuvent être valorisés de façon indépendante.

Grâce au procédé selon l'invention, l'économie réalisée sur l'unique étape d'extraction est substantielle. Comme montré dans les exemples suivants, elle peut atteindre jusqu'à 40 %.

### EXEMPLES

Les exemples ci-après illustrent l'invention, sans en limiter la portée.

### Exemple 1 (non conforme à l'invention)

On considère un procédé de production d'éthanol à partir de la fermentation des sucres en C6 (hexoses) et des sucres en C5 (pentoses).

Le substrat utilisé est de la paille, prétraitée dans des conditions acides. Le substrat prétraité est ensuite neutralisé, puis introduit dans le réacteur pour être converti en éthanol par hydrolyse enzymatique et fermentation du glucose et du mannose (sucres à 6 atomes de carbones).

Le procédé traite 52 tonnes / heure de pâte (base matière sèche). La composition de la matière sèche est la suivante:

| | |
|---|---|
| Cellulose (%) | 41.7% |
| Xylanes (%) | 25.2 % |
| Mannanes (%) | 0 % |
| Lignine (%) | 23.2 % |
| Autres (%) | 9.9 % |

Lors du prétraitement, les pertes de cellulose et de hémicelluloses sont de l'ordre respectivement de 5 et 10 %. A la sortie du prétraitement, le taux de matière sèche est de 35%(p/v) .

Le procédé de conversion en éthanol des sucres C6 et C5 contient les étapes suivantes: hydrolyse enzymatique, fermentation éthylique des sucres C6, séparation des résidus solides du vins, distillation de l'éthanol, fermentation éthylique de la coupe vinasse puis distillation de l'éthanol. Les deux étapes de distillation de l'éthanol peuvent être regroupées pour permettre de n'avoir qu'une seule colonne de rectification de l'éthanol.

L'hydrolyse enzymatique s'opère à pH 5, avec un flux d'entrée contenant 11,8% de matière sèche. Dans les conditions d'hydrolyse choisies, 95% des polymères de sucres sont solubilisés en monomères.

Le jus sucré est ensuite envoyé en fermentation éthylique où 90% des sucres glucose et mannose sont convertis en éthanol par *Saccharomyces cerevisae.*

Le vin est envoyé vers une centrifugeuse pour séparer les phases solide et liquide.

La phase liquide est ensuite envoyée en distillation. Le produit de tête contient majoritairement la fraction éthanol; le produit en fond contient les vinasses (mélange d'eau, de pentoses et les solides encore présents).

Les vinasses sont envoyées vers l'étape de fermentation éthylique réalisée par une levure de l'espèce *Pichia stipitis.* Les pentoses sont respectivement convertis en éthanol, sous produits et micro-organismes pour respectivement de 74,4, 24,5 et 1,1 % molaire.

En absence de recyclage, la fraction éthanol récupérée en tête de la distillation est envoyée vers une colonne de rectification, pour donner un mélange éthanol-eau à l'azéotrope.

Les produits issus de la fermentation éthylique des pentoses sont envoyés vers une colonne de distillation, qui va séparer l'eau puis vers une colonne de rectification (qui peut être la même que pour l'éthanol issu de la fermentation des hexoses), pour donner un mélange éthanol-eau à l'azéotrope.

Pour ce schéma de procédé, la production éthanol est de 14.6 t/h.

La consommation énergétique globale du procédé est de 34.6 MW ce qui correspond à une consommation moyenne de 8.55 MJ/kg d'éthanol produit

### Exemple 2

L'exemple 2 reprend les hypothèses de l'exemple 1 mais se distingue par un recyclage du flux post-fermentation des pentoses en deux points du procédé.

Le premier recyclage, correspondant à 71 % de ce flux, est envoyé en amont de l'hydrolyse enzymatique; le reste est envoyé vers la séparation commune éthanol post séparation des vins. L'appoint d'eau supplémentaire est nul au niveau de l'hydrolyse enzymatique.

La fraction d'éthanol récupérée en tête de la colonne des distillations des vins est envoyée vers une colonne de rectification pour séparer l'eau de l'éthanol (mélange éthanol-eau à l'azéotrope).

Pour ce schéma de procédé, la production éthanol est 14.46 t/h.

La consommation énergétique globale du procédé est de 30.3 MW ce qui correspond à une consommation moyenne de 7.55 MJ/kg d'éthanol produit.

Suivant l'invention, cet exemple utilisant un schéma avec deux recyclages éthanol permet un gain énergétique de 12 % par rapport à l'exemple 1 (MJ/kg d'éthanol produit).

### Exemple 3

L'exemple 3 reprend les hypothèses de l'exemple 1 mais se distingue par un recyclage partiel du flux post-fermentation des pentoses en amont de l'hydrolyse enzymatique. La partie du flux non recyclée est utilisée pour d'autres applications.

Le recyclage, correspondant à 71 % de ce flux, est envoyé en amont de l'hydrolyse enzymatique. L'appoint d'eau supplémentaire est nul au niveau de l'hydrolyse enzymatique.

La fraction d'éthanol récupérée en tête de la colonne des distillations des vins est envoyée vers une colonne de rectification pour séparer l'eau de l'éthanol (mélange éthanol-eau à l'azéotrope).

Pour ce schéma de procédé, la production d'éthanol est de 13.11 t/h.

La consommation énergétique globale du procédé est de 27.1 MW ce qui correspond à une consommation moyenne de 7.46 MJ/kg d'éthanol produit.

Dans cette configuration, le nombre d'équipement nécessaire au procédé est réduit d'une colonne de distillation.

Pour cet exemple, le schéma avec recyclage de l'éthanol issu de la fermentation des pentoses permet un gain d'équipement et un gain énergétique jusqu'à 13 % par rapport à l'exemple 1 (MJ/kg d'éthanol produit).

## Revendications

1. Procédé de production d'éthanol à partir de biomasse cellulosique ou lignocellulosique comprenant au moins
a) une étape de prétraitement thermo-chimique d'un substrat cellulosique ou lignocellulosique
b) éventuellement une étape de lavage du substrat prétraité, et de mise au pH
c) une étape d'hydrolyse enzymatique du substrat prétraité, éventuellement lavé, mettant en oeuvre des enzymes cellulolytiques et/ou hémicellulolytiques produisant un hydrolysat et un résidu insoluble dans l'eau ;
d) une étape de fermentation éthylique des hexoses contenus dans l'hydrolysat issu de l'étape c) en éthanol par un microorganisme alcooligène et obtention d'un vin éthylique ex-hexoses ;
e) une étape d'extraction comprenant
e1) la séparation et la purification de l'éthanol issus de l'étape d)
e2) la séparation d'un gâteau solide contenant le résidu insoluble et obtention de vinasses qui contiennent les pentoses non fermentés;
f) une étape de fermentation éthylique des pentoses contenus dans au moins les vinasses par un microorganisme fermentant les pentoses et obtention d'un vin éthylique ex-pentoses ;
dans lequel
- au moins une partie du vin éthylique ex-pentoses obtenu à l'étape f) est recyclée en amont d'au moins une des étapes d'hydrolyse enzymatique et/ou de fermentation alcoolique.

2. Procédé selon la revendication 1 dans lequel les pentoses fermentés à l'étape f) proviennent à la fois des vinasses et d'un flux directement extrait après l'étape de prétraitement lorsque celui-ci est acide.

3. Procédé selon l'une des revendications précédentes dans lequel la souche utilisée lors de l'étape f) est une souche de type *Pichia stipitis ou Candida shehatae.*

4. Procédé selon l'une des revendications précédentes dans lequel les étapes d'hydrolyse enzymatique et de fermentation éthylique des hexoses sont réalisées simultanément à une température comprise entre 30 et 45°C, à pH compris entre 4 et 6.

5. Procédé selon l'une des revendications précédentes dans lequel l'étape de séparation e2) du gâteau est réalisée en aval des étapes c) et/ou d) et est éventuellement couplée à un lavage du gâteau.

6. Procédé selon l'une des revendications précédentes dans lequel les vinasses envoyées vers l'étape de fermentation f) ont une concentration en sucres comprise entre 30 et 90g/L de sucres ou produits fermentescibles.

7. Procédé selon l'une des revendications précédentes dans lequel une partie du vin éthylique ex-pentoses est envoyé en aval de l'étape de prétraitement .

8. Procédé selon l'une des revendications 1 à 7 dans lequel seule une partie des vinasses est envoyée vers l'étape de fermentation éthylique des pentoses.

## Patentansprüche

1. Verfahren zur Erzeugung von Ethanol aus Zellulose- oder Lignozellulose-Biomasse, umfassend mindestens
a) einen Schritt der thermochemischen Vorbehandlung eines Zellulose- oder Lignozellulose-Substrats,
b) eventuell einen Schritt des Waschens des vorbehandelten Substrats und des Einstellens des pH-Werts,
c) einen Schritt der enzymatischen Hydrolyse des vorbehandelten, eventuell gewaschenen Substrats, der zellulolytische und/oder hemizellulolytische Enzyme einsetzt, die ein Hydrolysat und einen in Wasser nicht löslichen Rückstand erzeugen;
d) einen Schritt der Ethyl-Fermentation der Hexosen, die in dem Hydrolysat, das aus Schritt c) stammt, enthalten sind, zu Ethanol durch einen alkoholerzeugenden Mikroorganismus und Erhalt eines Ethylweins ohne Hexosen;
e) einen Extraktionsschritt, umfassend
e1) die Abscheidung und Reinigung des aus Schritt d) stammenden Ethanols,
e2) die Abscheidung eines Feststoffkuchens, der den unlöslichen Rückstand enthält, und Erhalt von Vinassen, die die nicht fermentierten Pentosen enthalten;
f) einen Schritt der Ethyl-Fermentation der zumindest in den Vinassen enthaltenen Pentosen durch einen die Pentosen fermentierenden Mikroorganismus und Erhalt eines Ethylweins ohne Pentosen;
bei dem
- mindestens ein Teil des Ethylweins ohne Pentosen, der in Schritt f) erhalten wird, stromaufwärts zu mindestens einem der Schritte der enzymatischen Hydrolyse und/oder der alkoholischen Fermentation wiederverwertet wird.

2. Verfahren nach Anspruch 1, bei dem die in Schritt f) fermentierten Pentosen von den Vinassen als auch von einem Strom stammen, der direkt nach dem Vorbehandlungsschritt entnommen wird, wenn dieser sauer ist.

3. Verfahren nach einem der vorhergehenden Ansprüche, bei dem der in Schritt f) verwendete Stamm ein Stamm vom Typ *Pichia stipitis oder Candida shehatae* ist.

4. Verfahren nach einem der vorhergehenden Ansprüche, bei dem die Schritte der enzymatischen Hydrolyse und der Ethyl-Fermentation der Hexosen gleichzeitig bei einer Temperatur zwischen 30 und 45 °C bei einem pH-Wert zwischen 4 und 6 durchgeführt werden.

5. Verfahren nach einem der vorhergehenden Ansprüche, bei dem der Schritt der Abscheidung e2) des Kuchens stromabwärts zu den Schritten c) und/der d) erfolgt und eventuell mit einem Waschen des Kuchens gekoppelt ist.

6. Verfahren nach einem der vorhergehenden Ansprüche, bei dem die zum Fermentationsschritt f) geschickten Vinassen eine Zuckerkonzentration zwischen 30 und 90 g/L Zucker oder fermentierbaren Produkten haben.

7. Verfahren nach einem der vorhergehenden Ansprüche, bei dem ein Teil des Ethylweins ohne Pentosen stromabwärts zum Vorbehandlungsschritt geschickt wird.

8. Verfahren nach einem der Ansprüche 1 bis 7, bei dem nur ein Teil der Vinassen zum Schritt der Ethyl-Fermentation der Pentosen geschickt wird.

## Claims

1. A process for the production of ethanol from a cellulosic or lignocellulosic biomass, comprising at least:
a) a step for thermochemical pre-treatment of a cellulosic or lignocellulosic substrate;
b) an optional step for washing the pre-treated substrate and for adjusting the pH;
c) a step for enzymatic hydrolysis of the pre-treated substrate, optionally washed, using cellulolytic and/or hemicellulolytic enzymes producing a hydrolysate and a water-insoluble residue;
d) a step for ethanolic fermentation of the hexoses contained in the hydrolysate obtained from step c) into ethanol by an alcoholigenic microorganism and for obtaining an ex-hexose ethanolic liquor;
e) an extraction step comprising:
e1) separating and purifying the ethanol obtained from step d);
e2) separating a solid cake containing the insoluble residue and obtaining stillage that contain-non fermented pentoses;
f) a step for ethanolic fermentation of the pentoses contained in the stillage with a pentose-fermenting microorganism and for obtaining an ex-pentose ethanolic liquor;
in which at least a portion of the ex-pentose ethanolic liquor obtained in step f) is recycled upstream of at least one of the enzymatic hydrolysis and/or alcoholic fermentation steps.

2. A process according to claim 1, in which the pentoses fermented in step f) originate both from the stillage and from a stream withdrawn directly after the pre-treatment step when this latter is acid.

3. A process according to one of the preceding claims, in which the strain used in step f) is a strain of the *Pichia stipites* or *Candida shehatae* type.

4. A process according to one of the preceding claims, in which the enzymatic hydrolysis and ethanolic fermentation of the hexoses steps are carried out simultaneously at a temperature in the range 30°C to 45°C at a pH in the range 4 to 6.

5. A process according to one of the preceding claims, in which the cake separation step e2) is carried out downstream of steps c) and/or d) and is optionally coupled with washing the cake.

6. A process according to one of the preceding claims, in which the stillage sent to the fermentation step f) has a sugar concentration in the range 30 to 90 g/L of sugars or fermentiscible products.

7. A process according to one of the preceding claims, in which a portion of the stillage is sent downstream of the pre-treatment step.

8. A process according to one of claims 1 to 7, in which only a portion of the stillage is sent to the pentose ethanolic fermentation step.
